# EUROPEAN PATENT SPECIFICATION

(11) **EP 3 327 602 B1**
(45) Date of publication and mention of the grant of the patent: **30.12.2020**
(21) Application number: 17208306.5
(22) Date of filing: 20.06.2008
(51) Int. Cl.: G16H 10/60

(54) **METHOD FOR DERIVING HEALTH PROFILES FROM INFORMATION OBTAINED FROM ORAL CARE IMPLEMENTS**
VERFAHREN ZUM ABLEITEN VON GESUNDHEITSPROFILEN AUS VON MUNDPFLEGEVORRICHTUNGEN GEWONNENEN INFORMATIONEN
PROCÉDÉ POUR DÉRIVER DES PROFILS DE SANTÉ À PARTIR D'INFORMATIONS OBTENUES À PARTIR D'ARTICLES D'HYGIÈNE BUCCALE

(30) Priority: 19.06.2008 US 142136
(43) Date of publication of application: 30.05.2018
(62) Document number(s) of the earlier application(s) in accordance with Art. 76 EPC: 08771554.6 / 2 307 995
(73) Proprietor: Colgate-Palmolive Company, New York, NY 10022 (US)
(72) Inventor: GATZEMEYER, John, J., Hillsborough, New Jersey 08844 (US); GITTINS, Elizabeth, Stewartsville, New Jersey 08886 (US); JIMENEZ, Eduardo, Manalapan, New Jersey 07726 (US); KENNEDY, Sharon, Randallstown, Maryland 21133 (US); TRIVEDI, Harsh, M., Hillsborough, New Jersey 08844 (US)
(74) Representative: Schicker, Silvia

(56) References cited:
- EP-A- 1 698 252
- WO-A-2006/137661
- DE-A1- 10 045 067
- US-A1- 2002 127 143
- US-B1- 7 386 333

## Description

Tooth brushing is part of a daily oral hygiene activity, Proper dental care involves regular flossing, brushing and dental checkups. According to the American Dental Association, a major source of bad breath in healthy people is microbial deposits on the tongue, where a bacterial coating harbors organisms and debris that contribute to bad breath. Tissue in the mouth, and especially the tongue, is a haven for the growth of microorganisms, Anaerobic flora and bacteria residing on the tongue and other soft tissues in the mouth play a role in the development of chronic bad breath commonly called halitosis. In general, the bacteria produce volatile sulfur compounds (VSC). If there is enough buildup of the sulfur compounds, the result can be bad breath or oral malodor.

Dentists generally recommend that an individual brush his or her teeth for a minimum interval per cleaning, such as two minutes. Despite such recommendations, many individuals, especially young children, do not regularly brush their teeth for the recommended minimum interval. Such habits often can be attributed to the individual regarding tooth brushing as a mundane duty with few pleasurable aspects. Dental checkups to a dentist office are typically the only feedback a person receives on their dental cleaning efforts, There is unfortunately ample time between checkups for poor dental habits to cause problems. For example, gingivitis, periodontal disease, and a host of other problems can be magnified if poor dental cleaning habits are not rectified promptly.

EP 1 698 252 A2 describes a toothbrush comprising a sensor for collecting brushing data, a microprocessor and a data storage device. Also described is a method which acquires the data indicative of motion of the toothbrush and pressure applied thereon, analyzes the data to determine the brushing motion of the toothbrush and output the data to a user on a display of the toothbrush or a larger screen, such as of a computer or a toothbrush holder where the data can be broken down by region of the mouth.

WO 2006/137661 A1 describes a method for managing oral health in which a user may be guided to acquire correct toothbrushing habits. In the method, toothbrushing pattern data is received from a user's correction toothbrush when in use and is recorded in a toothbrushing pattern database. The recorded toothbrushing pattern data is analyzed, the user's adaptability is determined according to the analyzed results and toothbrushing correction multimedia contents are chosen and fed back to the user in which the adaptability of the user is evaluated, wherein the feedback includes toothbrushing habits, toothbrushing period, toothbrushing pattern of each teeth region, etc.

DE 100 45 067 A1 describes a system for supporting a user in performing care measures in dental area. The system comprises a processing unit for controlling the system, visual and acoustic reproducing means, and a storage device containing data for instructing the user on how to perform care measures in dental area. The user is provided via a monitor and/or a speaker with instructions, such as displaying dental regions to be cleaned. The care measures can be optimized by adjusting the data in the storage device to the user by using an oral care system unit which comprises a transmission unit, a position sensor and/or a pressure sensor, wherein the sensors are configured to record location, position and/or orientation of the oral care system unit and/or forces applied on the used oral care system unit and the transmission unit is configured to transfer this data from the sensors to the processing unit of the system. The system further provides a user interface for interactively controlling the system and making user-specific adjustments thereto.

US 7 386 333 B1 describes a device for monitoring a test subject including a light source, a plurality of optical waveguides configured for directing the light from the light source to a biological substrate and receiving the light directed onto the biological substrate, wherein the device generally comprises a toothbrush and the biological substrate may be a sample of blood or saliva. Furthermore, the device includes a sensor operable to detect the light received, a logic component operable to analyze the detected light, the output component to convey information from logic component to a user of the device and a housing containing the light source, the sensor, the logic component and output component, where the at least one of the optical waveguides forms a bristle extending outwardly from the housing.

### BRIEF SUMMARY OF THE INVENTION

Aspects of the present invention are directed to providing an oral care implement (such as a toothbrush) having an oral care diagnostic feature. Various aspects of the oral care implement may improve dental care by providing feedback on a user's brushing efforts.

Further aspects are directed to providing a health profile to the user based on information from such a diagnostically-capable oral care implement, or a conventional oral care implement. The health profile may be created by analyzing information obtained from the oral care implement and presenting representations of certain health aspects. The health aspects may include indications relating, for example, to whether certain oral or non-oral diseases are suspected, as well as to whether certain brushing techniques are being properly executed. This type of health profile may, in certain circumstances, provide even more information to a user than may be available via feedback provided by the oral care implement itself.

Still further aspects are directed to providing a health profile service business, such as over the Internet and/or through a brick-and-mortar boutique storefront. In such a case, data from the oral care implement, or even the oral care implement itself, may be provided to the health profile service. The service may in turn process the data and/or oral care implement to generate the health profile, which is then provided to the customer/user of the oral care implement.

### BRIEF DESCRIPTION OF THE DRAWINGS

Fig. 1 is a side, cross-sectional view of a top portion of an illustrative toothbrush having one or more sensors.
Fig. 2 is an illustrative functional block diagram of the toothbrush of Fig. 1.
Fig. 3 is a functional block diagram of an illustrative system and process for providing a health profile of a customer from data gathered from the customer's toothbrush.
Fig. 4 is a flow chart of illustrative steps that may be performed to provide the health profile.
Fig. 5 is an illustrative screen shot of a displayed web page providing representation of the health profile.

### DETAILED DESCRIPTION OF THE INVENTION

The following detailed description refers to the accompanying drawings. The same reference numbers in different figures identify the same or similar elements.

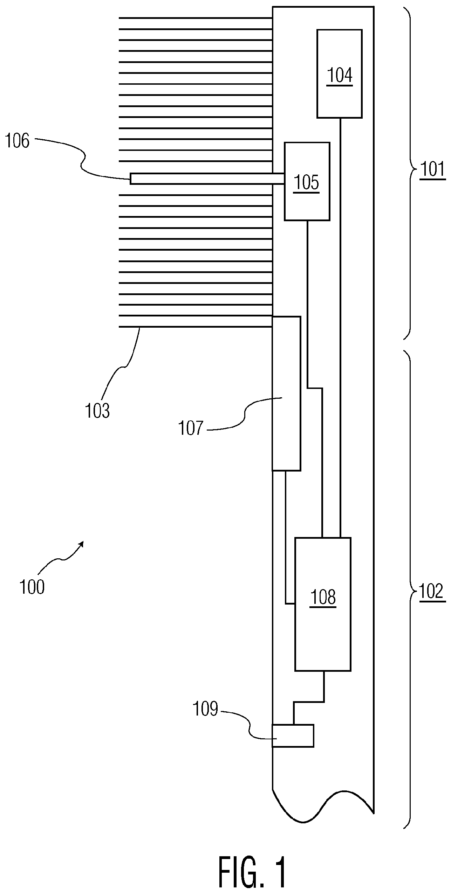

Fig. 1 illustrates the top portion of an illustrative oral care implement, such as a toothbrush 100. As illustrated, the toothbrush 100 has a head 101 and a handle 102. The head 101 may include an oral care region comprising one or more tooth cleaning elements 103, such as bristles. The head 101 and handle 102 may be made from any desired material, such as rigid plastics, resins, rubber, etc., such as polypropylene.

As used herein, the term "tooth cleaning element" or "cleaning element" includes any type of structure that is commonly used or is suitable for use in providing oral health benefits (e.g., tooth cleaning, tooth polishing, tooth whitening, massaging, stimulating, etc.) by making contact with portions of the teeth and gums. Such tooth cleaning elements include but are not limited to tufts of bristles that can be formed to have a number of different shapes and sizes and elastomeric cleaning members that can be formed to have a number of different shapes and sizes, or a combination of both tufts of bristles and elastomeric cleaning members.

The toothbrush 100 may include a number of additional components to provide features for improved oral care. For example, the toothbrush 100 may include a position sensor 104. The position sensor 104 may be any desired device that can be used to detect a position (or orientation) of the toothbrush 100. For example, the position sensor 104 may be a single- or multi-axis (e.g., 2-D or 3-D) accelerometer, and may be of any desired accelerometer type (e.g., piezoelectric, capacitive or inductive, optical, etc.). The position sensor 104 may be a single, unitary component, or it may include multiple devices, such as multiple accelerometers placed at different orientations. The position sensor 104 may indicate, for example, the angular position with which the user is holding the toothbrush (e.g., 45 degrees with respect to the horizon). The position sensor 104 may also indicate, for example, whether the toothbrush is being held bristle-side up or bristle-side down.

In one illustrative construction, the position sensor 104 may be an accelerometer utilizing Micro-Electronic Mechanical System (MEMS) technology. The position sensor 104 may be embedded in the toothbrush during a molding of toothbrush manufacturing, or may be applied at a later time such as by being in the form of a sticker applied on an exterior of the toothbrush body. A MEMS accelerometer device generates a signal representative of acceleration in a particular direction ("measurement direction"). The MEMS accelerometer detects gravitational force when not being accelerated. Accordingly, different altitudes of the MEMS accelerometer device with respect to the vertical can result in different readings which depend on the coincidence of the measurement direction with the direction of the gravitation pull. The detection of different altitudes may be used to detect the position of the toothbrush and the relative change in position from a first state to a second state, such as from bristle-side to bristle-side down or vice-versa.

The toothbrush 100 may also include a diagnostic sensor or bio-sensor 105. The diagnostic sensor 105 may include one or more individualized sensors or sensor suites to detect various characteristics of a sampled liquid or vapor. For example, the diagnostic sensor 105 may include a sensor configured to detect the pH of the sample, the electrical conductivity or resistivity of the sample, the presence of bacteria in the sample, the presence of certain chemicals in the sample, or any other desired characteristic. These components of the diagnostic sensor 105 may work together to sense whether the sample contains evidence of halitosis, gingivitis, periodontitis, plaque, or any other undesired characteristic. The sensed characteristics need not all be undesirable, either. For example, the diagnostic sensor 105 may be configured to detect the presence of flavanoid alcohols, oils or flavors, which may be indicative of the user having fresh or "good" smelling breath, or that the user has sufficiently brushed with a proscribed dentrifice.

In one construction, the diagnostic sensor 105 is configured to obtain a liquid or vapor sample for its sensing function. For example, the toothbrush 100 may include one or more channel bristles 106, which may be hollow tubes (e.g., including a lumen) that allow liquid and/or vapors to pass, such as oral fluids (e.g., salvia or breath). The channel bristles 106 may be coupled to the diagnostic sensor 105 to provide the sample. The channel bristles 106 may rely simply on capillary action and the motion of brushing to convey the sample, or the toothbrush 100 may include pumps and/or other structures (not illustrated) to draw samples in and to purge the samples when the sensing is completed. Alternative configurations can be used as well. For example, the diagnostic sensor 105 may simply have a portion exposed directly to an outer surface of the toothbrush 100, such that sampling can occur simply by the toothbrush 100 coming into contact with the liquid and/or vapor to be sampled. One example of a commercially available diagnostic sensor using MEMS microfluidics technology is provided by STMicroelectronics of Geneva Switzerland, such as its IN-CHECK platform. This particular system is based on a silicon chip that integrates various functions including microfluidic handling, a miniaturized Polymerase Chain Reaction (PCR) reactor and a microarray. As one skilled in art would understand, PCR can be used to detect oral care pathogens linked to halitosis, gingivitis, and periodontitis, for example. Nevertheless, other diagnostic sensors can be used, such as a pH sensor.

The toothbrush 100 may also include one or more pressure sensors 107. The pressure sensor 107 may be configured to detect the amount of pressure or force that the user is using when brushing his/her teeth. Any type of pressure sensor 107 may be used. For example, a stress/strain type of piezoelectric sensor may be placed at a neck portion (an area where the head 101 and handle 102 meet) to measure how far the head 101 bends during brushing.

The various sensors 104, 105, 107 described above may be electrically or communicatively coupled to a processor 108. The processor 108 generally handles the information gathered by the sensors 104, 105, 107, and reports an analysis of that information to the user, to help inform the user of the conditions within his/her mouth. For example, the toothbrush 100 processor 108 may report that it has detected a higher than normal level of plaque in the upper-left portion of the user's teeth. Additional details of the processor 108, and its operation, are found further below.

The toothbrush 100 may also include one or more input/output (I/O) interfaces 109. The I/O interface 109 may contain any type of electrical interface used to provide data to, or retrieve data from, the processor 108. The I/O interface 109 may be a plug-in physical connection, such as those used for radio headsets, Universal Serial Bus (USB), IEEE-1319 Fire Wire, or any other wired interface. Alternatively, or additionally, the interface 109 may include wireless capabilities, such as Bluetooth or IEEE 802.11 wireless interfaces and associated components. In yet another construction, toothbrush 100 may be a ZIGBEE complaint device. ZIGBEE pertains to an industry specification for a suite of communication protocols using small, low-power digital radio based on the IEEE 802.15.4 standard for wireless personal area networks which is incorporated by reference herein. The radio-controlled configuration may include a transmitter and receiver operating at 2.4 GHz, but other frequencies may be implemented in different geographic regions. The data transfer rates can be 250 Kbs, but other data rates slightly lower or higher could be used. Various commercially available ZIGBEE compliant modules could be implemented. Other known wireless transmission protocols or wireless medium arrangements also can be employed. These interfaces may be used to transmit data, such as the reports discussed below, to an external computing device, such as a personal computer or an Internet web server, as will be discussed further below.

Other components may also be included on the toothbrush 100. For example, a digital display (not shown) may be placed on the toothbrush to assist in reporting output. The display may include light-emitting diode (LED) displays, an alphanumeric display screen, individual lights, or any other desired form of visual output. For example, the display may be an organic LED (or array thereof) that can be tuned to provide a desired luminescent characteristic such as color, temperature, intensity etc. OLED technology can be embedded into the toothbrush molding, or can be applied to the surface of the toothbrush body. It should be understood by those skilled in the art that the present invention is not limited to any particular type of display.

The toothbrush 100 may also include one or more speakers (not shown), to allow the processor 108 to generate audible sounds and tones.

A power source (not shown) for the processor 108 and sensors may also be included. The power source may simply be one or more batteries in a lower portion of the handle 102. Alternative sources, such as solar, motion-based, thermal, etc., may also be used.

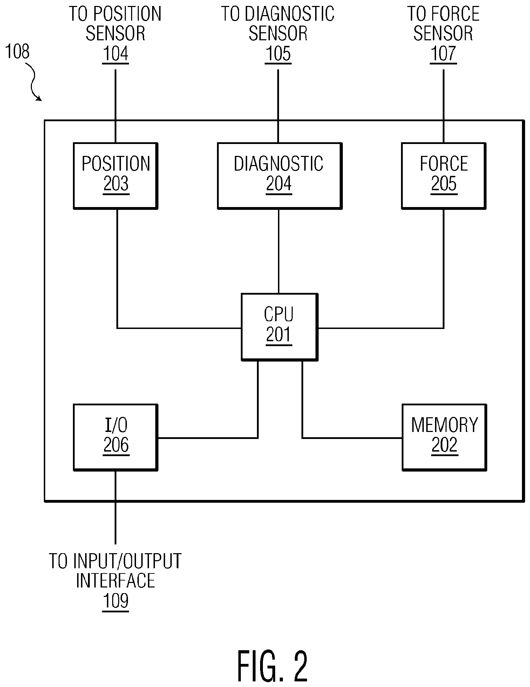

Fig. 2 illustrates example components of the processor 108. The processor 108 may be a single integrated circuit (e.g., an application-specific integrated circuit), multiple interconnected circuits, and/or hard-wired circuit logic to perform as described herein. Similarly, the individual components depicted in Figure 2 may also be integrated, separated or hard-wired as well. The processor 108 may include a central processing unit (CPU) 201, which may be a programmable device or dedicated logic circuit or integrated circuit. The CPU 201 may operate with a memory 202, which may be any type of recordable, computer-readable media. For example, memory 202 may be a static flash memory, dynamic memory, or any other type of electronic storage. In some embodiments, the CPU 201 is a general programmable device, and the memory 202 stores instructions that cause the CPU 201 to perform the various functions described herein.

Those functions include, for example, obtaining information from the various sensors. For example, the processor 108 may have a position sensor interface 203, through which the CPU 201 may communicate with the position sensor 104. The interface may, for example, simply be a pin on an integrated circuit, a wire, or any other desired type of interface, and may vary depending on the type of position sensor chosen. Similarly, the processor 108 may have an I/O interface 206, which can serve as an interface through which the CPU 201 receives and/or transmits data. The lines illustrating wire connections between these components need not be literally as depicted, and alternative interconnections may be used.

As mentioned previously, the data output from toothbrush 100 by I/O interface 206 may be provided to an external computing device. This computing device (or a system of computing devices) may use this data to create a health profile of the user of toothbrush 100.

A health profile includes an indication of one or more aspects of the health of an individual. The health aspects may be related to oral health, such as whether the individual has or is suspected of having gingivitis, periodiontal gum disease, plaque, and/or halitosis and the like. The health aspects may additionally or alternatively be related to oral-related health indicators, such as whether the individual is, or is suspected of, or is at increased risk of, having heart disease, such as based on the presence of pathogens in the individual's mouth, and/or the individual's body temperature. The health aspects may additionally or alternatively be related to oral care events relating to the use of the toothbrush itself, such as whether the toothbrush is being used properly. For instance, some health aspects may indicate whether a toothbrush was applied with excessive pressure to the teeth or gums, whether the toothbrush was used for a sufficient time in a brushing episode, and/or whether the brushing efficiency was thorough or uneven.

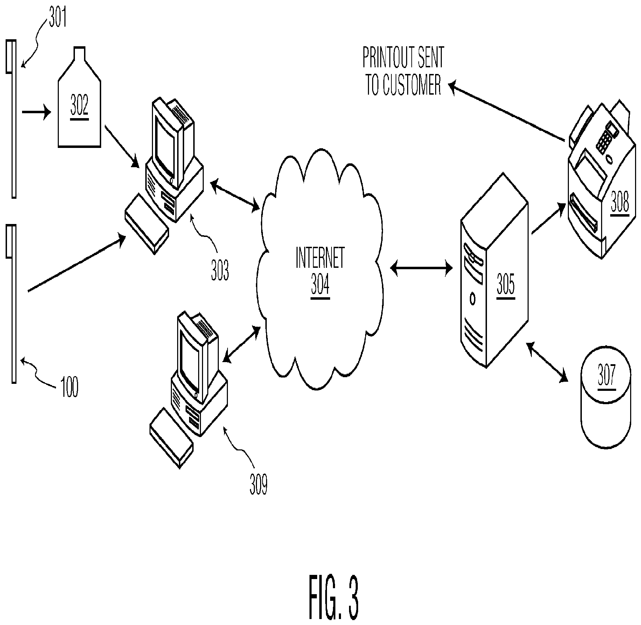

Referring to Fig. 3, a functional block diagram of an illustrative system and process for providing a health profile of a customer from data gathered from the customer's toothbrush is shown. In this example, a system for providing a health profile may include a first computing device 303, a second computing device 305, and/or a third computing device 309. In addition, one or more of first, second, and third computing devices 303, 305, 309 may include or be coupled with a storage device and/or a printer. In the example shown, computing device 305 is coupled with a storage device 307 and a printer 309.

First, second, and third computing devices 303, 305, 309 may each be any type of computing device, such as one or more laptop computers, desktop computers, personal digital assistants, servers, and/or cellular phones. Each of computing devices 303, 305, 309 may also include a communication interface for sending and/or receiving data to/from a location external to the respective computing device. For example, computing devices 303, 305, 309 may be capable of communicating data back and forth between each other such as via the Internet 304, another type of network such as a cellular and/or landline telephone network, and/or via a direct wired and/or wireless connection.

In operation, information about a toothbrush or other type of oral care implement may be provided to first computing device 303. Two alternative scenarios are shown in Fig. 3. In a first scenario, toothbrush 100 is configured such as shown in Fig. 1. In other words, toothbrush 100 has sensors and a means for outputting data from those sensors. In this case, data may be retrieved from toothbrush 100 (e.g., from I/O interface 206) and sent directly to first computing device 303.

In a second scenario, a toothbrush 301 may be provided that does not have any data output capability. For example, toothbrush 301 may be a conventional toothbrush. In such a case, a chemical and/or biological physical sample may be taken from toothbrush 301 and provided to an analysis processor 302 for analyzing the sample. The sample may include, for example, a portion of toothbrush 301 (or in fact the entirety of toothbrush 301), or a residue disposed on toothbrush 301 that may be removed from toothbrush 301 by any of various means such as by wiping or by dissolving using a solvent. Analysis processor 302 may include, for instance, a silicon chip that integrates the functions of microfluidic handling, a miniaturized PCR reactor, and a microarray. As previously mentioned, PCR can be used to detect oral care pathogens linked to halitosis, gingivitis, and periodontitis, for example. Other diagnostic sensors can additionally or alternatively be used in analysis processor 302, such as a pH sensor. Regardless of how the sample is analyzed in processor 302, the results of processor 302 may be entered (manually or automatically) into first computing device 303.

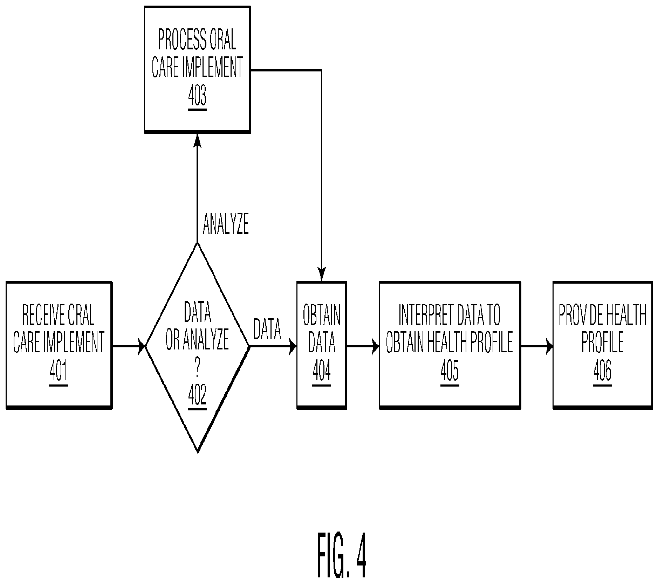

Referring now to Fig. 4, a flow chart of an illustrative method for providing a health profile of a customer is shown. An oral care implement (such as toothbrush 100 or toothbrush 301) is provided to and used by a customer in step 401. The oral care implement may either be used by the customer at his home, or the customer may go to a service provider where the customer is given the oral care implement to use at the service provider's site. At step 402, the oral care implement is transferred from the customer and received by the service provider. It is determined in step 403 whether the oral care implement is of a type that can provide data or of a type that should be analyzed using processor 302. In either case, data representing information obtained from the toothbrush is inputted to first computing device 303 in step 405.

Returning to the example of Fig. 3, first computing device 303 may process the input data and provide the information represented by the data to second computing device 305 via the Internet 304. Then, second computing device 305 may interpret the received data to create a health profile (step 406 in Fig. 4). The health profile may additionally be stored, such as in storage device 307. Storage device 307 may be any one or more devices suitable for storing data, such as semiconductor memory, hard drives, magnetic tape, optical drives, etc.

Next, the health profile may be output or otherwise made available to a user (step 407 in Fig. 4), such as by displaying a representation of the health profile on a computer display, by providing a displayed representation of the health profile on a website that is made available via the Internet 304, by printing a representation of the health profile on printer 308, and/or by emailing a representation of the health profile such as to the user of toothbrush 100 or 301. Where a website provides the health profile, the website may be accessed and viewed by any authorized device such as third computing device 309 running a web browser. Where an email is sent, the email may also be received by third computing device 309.

The present example may be further expanded into a method of providing oral health services. More specifically, a health profile service provider can cater to the public consumer by providing the health profile. For example, computing devices 303 and 305, printer 308, storage device 307, and processor 302 may be owned by or otherwise under the control of the service provider. The toothbrush 100/301 and computing device 309 may be owned by or otherwise under the control of either the service provider or a customer of the service provider. It can be envisioned that such a service provider may provide one or more brick-and-mortar boutiques to which customers may come to use or bring the toothbrushes or other oral care implements for processing and obtaining health profiles. Alternatively, if the toothbrush includes a data output capability, such as toothbrush 100, the customer may provide the data rather than the physical toothbrush for processing. In addition to or as an alternative to brick-and-mortar boutique storefronts, the service provider may operate over the Internet or by mail order such that the customer may upload data via the Internet to the remote business entity, or send the toothbrush to the business entity through the mail.

Alternatively, a private user may own or otherwise exercise control over computing device 303, and also over processor 302. In that context (and in some circumstances in the previously-described service provider context), steps 406 and 407 may be performed by computing device 303 such that the remaining elements 304-309 are not needed.

In general, any of the steps in Figs. 3 and 4 may be implemented by or assisted by one or more of computing devices 303, 305, and/or 309, such as by programming or otherwise configuring these computing devices with software in the form of computer-executable instructions and/or data stored on a computer-readable medium. The computer-readable medium may include the storage media of any one or more storage devices (such as storage device 307 and/or storage devices inherent to computing devices 303, 305, and 309). As a non-limiting example, a computer-readable medium may include one or more of any of the following in any combination: a computer memory chip, a magnetic disc or tape, or an optical disc.

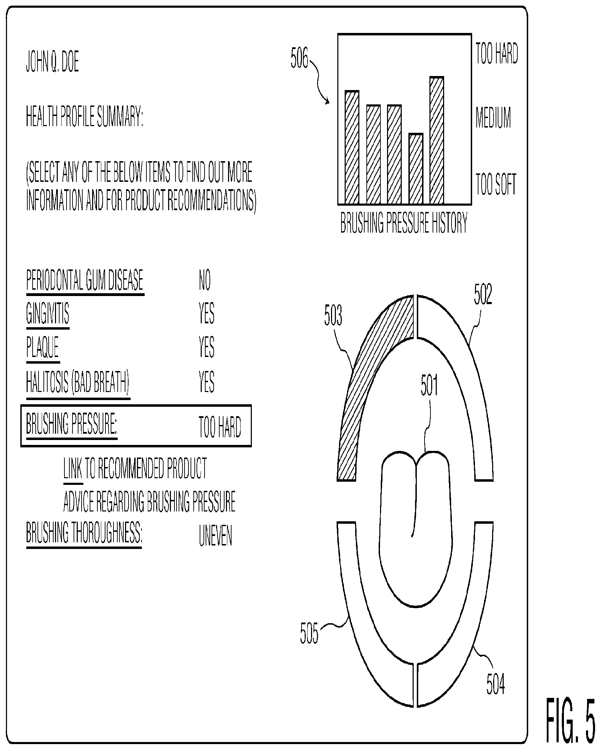

Fig. 5 shows an example of a displayed representation of a health care profile. This displayed content (i.e., the displayed representation) may be provided by any of computing devices 303, 305, or 309. In some embodiments, the displayed content may be provided on a website or in an email. Where the displayed content is provided on a website, for example, the website may be interactive such that the displayed content changes in response to user selections.

In the example shown in Fig. 5, the displayed content includes an identification of the toothbrush user and/or customer (in this case, "John Q. Doe"), and indications of various aspects of the health profile, including whether or not the following conditions have been detected based on information obtained from toothbrush 100 or 301: periodontal gum disease, gingivitis, plaque, and halitosis (bad breath). In addition, the indicated aspects further include in this example an indication of the brushing pressure experienced by the toothbrush, as well as an indication of the brushing thoroughness experienced by the toothbrush. It will be understood that these aspects are merely illustrative, and that any combination of these and/or other health profile aspects may be indicated. The indicated health aspects may include aspects related to bad health (e.g., the presence of a disease, such as indicated by the presence of a pathogen or the presence or absence of another chemical) and/or aspects related to good health (e.g., the presence of certain desirable flavanoids indicative of the activity of a breath freshener). The aspects of good health and bad health may include, but are not limited to, aspects of good and bad oral health. Bad oral health aspects may include, for example, those aspects associated with oral diseases or with poor oral care practices recognized or otherwise identified by the American Dental Association. Likewise, good oral health aspects may include, for example, those aspects associated with the lack of such oral diseases, or with oral care practices accepted by the American Dental Association.

The displayed content may further include a graphical depiction of an oral cavity divided into multiple different regions. This regional information may be alternatively provided textually rather than graphically. In this case, the various regions are the tongue 501, the upper left quadrant of teeth and/or gums 502, the upper right quadrant of teeth and/or gums 503, the lower left quadrant of teeth and/or gums 504, and the lower right quadrant of teeth and/or gums 505. Other regions may be defined as well. For example, the quadrants 502-505 may be further divided into smaller regions, and the tongue 501 may also be subdivided into regions. As another example, the may be only three regions: a tongue region, an upper teeth/gum region, and a lower teeth/gum region. In still another example, there may be no tongue region at all.

Where the display is interactive, the user may select any one of the displayed health aspects to learn more about the selected health aspect. Selection may be performed in any manner desired, such as by the user "clicking" a mouse pointer on the displayed health aspect to be selected, or by using a keyboard to control a selection cursor. In any event, selection of a displayed health aspect may cause the displayed content to provide additional information such as an indication of which of the regions 501-505 of the oral cavity is affected by the selected health aspect (such as by highlighting or otherwise visually distinguishing the affected oral cavity regions), or an indication of recommended products for addressing the health aspect or other advice for dealing with the health aspect. Such products may include, for example, a particular oral care implement or dentifrice. The indication of the particular regions 501-505 may be obtained from data originally generated by position sensor 104 in toothbrush 100, for example.

In the present example as shown in Fig. 5, the health aspect "brushing pressure" has been selected (indicated by the box displayed around this health aspect), which has been indicated as being presently at the "too hard" level (from a selection of "too hard," "medium," and "too soft"). As a result of the user selection, a user-selectable hyperlink to a product for sale that is recommended for assisting the user with proper brushing pressure may be displayed, and/ or advice on how to apply proper brushing pressure. It will be understood that user selection of any of the other displayed health aspects may result in a similar set of information customized to the particular selected health aspect.

Also as a result of the user selection of a particular health aspect, an indication of historical values associated with that health aspect may be displayed. For instance, a bar chart 506 or other graphical representation may display previous values or other indications of previous results for that health aspect for the particular user (in this case, for user John Q. Doe). To accomplish this, data representing each user's health profile may be stored (such as in storage device 307) and associated with the respective user, thereby accumulating a health profile history for each user.

In alternative embodiments, information about recommended products for sale, advice about dealing with a particular health aspects, indications of oral cavity regions, and/or health profile history may be provided regardless of user selection.

This same information shown on the display of Fig. 5 may be printed on paper by printer 308 and mailed or otherwise provided to the customer or other user of the toothbrush, and/or included in an email to the customer or other user.

While many examples have been discussed with respect to using a toothbrush (such as toothbrushes 100 and 301), the various features and steps described herein may be used in connection with any type of oral care implement having a tooth cleaning element.

## Claims

1. A system for providing a health profile of an individual, comprising:
a toothbrush (100) comprising data outputting means (206), wherein the toothbrush (100) has a tooth cleaning element (103) and a sensor (105), wherein the data outputting means (206) comprises means for outputting data from the sensor (105), and wherein the sensor is a diagnostic sensor (105);
a computing unit (303) configured to derive a health profile of an individual using data obtained from the data outputting means (206) of the toothbrush (100); and
a display configured to display a representation of the health profile,
wherein the representation of the health profile comprises an indication of whether the individual is suspected of having a health aspect such as one or more of gingivitis, periodontal gum disease, plaque, halitosis and heart disease, based on the data obtained;
**characterised in that**
the representation of the health profile includes a graphical depiction of an oral cavity divided into a plurality of different regions (501, 502, 503, 504, 505), wherein the plurality of different regions (501, 502, 503, 504, 505) comprise an upper teeth region and a lower teeth region, and wherein selection of one of the health aspects causes the representation of the health profile to provide an indication of which of the plurality of different regions (501, 502, 503, 504, 505) of the oral cavity is affected by the selected health aspect; and
**in that** the toothbrush (100) comprises a position sensor (104) operable to detect a position or an orientation of the toothbrush (100) so as to indicate whether the toothbrush (100) is being held bristle-side up or bristle-side down;
wherein the data outputting means (206) comprises means for outputting data from the position sensor (104); and
wherein the indication of which of the plurality of different regions (501, 502, 503, 504, 505) of the oral cavity which comprise an upper teeth region and a lower teeth region is affected by the health aspect is obtained from the data originally generated by the position sensor (104) which indicate whether the toothbrush (100) is being held bristle-side up or bristle-side down.

2. The system of claim 1, wherein the representation of the health profile of an individual is displayed based on first information obtained from the toothbrush (100), the representation further including a displayed indication of a history of the health profile of the individual based on second information previously obtained from the toothbrush (100).

3. The system of claim 1, wherein the data is received via the Internet (304).

4. The system of claim 1, wherein the display is separate from the toothbrush (100).

5. The system of any one of claims 1 to 4, wherein the representation of the health profile comprises an indication of a product recommended for addressing the health aspect.

6. The system of claim 5, wherein the product is an oral care implement.

7. The system of claim 5, wherein the product is a dentifrice.

8. The system of any one of claims 1 to 7, wherein the indication comprises highlighting or otherwise visually distinguishing the affected regions.

## Patentansprüche

1. System zum Bereitstellen eines Gesundheitsprofils einer Person, umfassend:
eine Zahnbürste (100), umfassend Datenausgabemittel (206), wobei die Zahnbürste (100) ein Zahnreinigungselement (103) und einen Sensor (105) aufweist, wobei die Datenausgabemittel (206) Mittel zum Ausgeben von Daten von dem Sensor (105) umfassen und wobei der Sensor ein Diagnosesensor (105) ist;
eine Recheneinheit (303), die konfiguriert ist, um ein Gesundheitsprofil einer Person unter Verwendung von Daten abzuleiten, die von dem Datenausgabemittel (206) der Zahnbürste (100) erhalten werden; und
eine Anzeige, die konfiguriert ist, um eine Darstellung des Gesundheitsprofils anzuzeigen,
wobei die Darstellung des Gesundheitsprofils eine Angabe darüber umfasst, ob basierend auf den erhaltenen Daten ein Verdacht besteht, dass die Person einen Gesundheitsaspekt aufweist, wie zum Beispiel eines oder mehrere von einer Zahnfleischentzündung, einer parodontalen Zahnfleischentzündung, Plaque, Mundgeruch und Herzkrankheit;
**dadurch gekennzeichnet, dass**
die Darstellung des Gesundheitsprofils eine graphische Darstellung eines Mundraums beinhaltet, der in eine Mehrzahl unterschiedlicher Bereiche (501, 502, 503, 504, 505) unterteilt ist, wobei die Mehrzahl unterschiedlicher Bereiche (501, 502, 503, 504, 505) einen oberen Zahnbereich und einen unteren Zahnbereich umfasst und wobei die Auswahl von einem der Gesundheitsaspekte bewirkt, dass die Darstellung des Gesundheitsprofils einen Hinweis bereitstellt, welcher aus der Mehrzahl verschiedener Bereiche (501, 502, 503, 504, 505) des Mundraums von dem ausgewählten Gesundheitsaspekt betroffen ist; und
dass die Zahnbürste (100) einen Positionssensor (104) umfasst, der betreibbar ist, um eine Position oder eine Ausrichtung der Zahnbürste (100) zu erkennen, um anzugeben, ob die Zahnbürste (100) mit der Borstenseite nach oben oder mit der Borstenseite nach unten gehalten wird;
wobei die Datenausgabemittel (206) Mittel zum Ausgeben von Daten von dem Positionssensor (104) umfassen; und
wobei die Angabe, welcher aus der Mehrzahl verschiedener Bereiche (501, 502, 503, 504, 505) des Mundraums, die einen oberen Zahnbereich und einen unteren Zahnbereich umfassen, von dem Gesundheitsaspekt betroffen ist, aus den Daten erhalten wird, die ursprünglich von dem Positionssensor (104) erzeugt werden, die angeben, ob die Zahnbürste (100) mit der Borstenseite nach oben oder mit der Borstenseite nach unten gehalten wird.

2. System nach Anspruch 1, wobei die Darstellung des Gesundheitsprofils einer Person basierend auf einer ersten Information, die von der Zahnbürste (100) erhalten wird, angezeigt wird, wobei die Darstellung ferner eine angezeigte Angabe über einen Verlauf des Gesundheitsprofils der Person basierend auf der zweiten Information, die vorab von der Zahnbürste (100) erhalten wurde, umfasst.

3. System nach Anspruch 1, wobei die Daten über das Internet empfangen werden (304).

4. System nach Anspruch 1, wobei die Anzeige von der Zahnbürste getrennt ist (100).

5. System nach einem der Ansprüche 1 bis 4, wobei die Darstellung des Gesundheitsprofils eine Angabe eines Produkts umfasst, das zur Behandlung des Gesundheitsaspekts empfohlen wird.

6. System nach Anspruch 5, wobei das Produkt ein Mundpflegeprodukt ist.

7. System nach Anspruch 5, wobei das Produkt eine Zahncreme ist.

8. System nach einem der Ansprüche 1 bis 7, wobei die Angabe das Hervorheben oder eine sonstige visuelle Unterscheidung der betroffenen Bereiche umfasst.

## Revendications

1. Système pour fournir un profil de santé d'un individu, comprenant :
une brosse à dents (100) comprenant un moyen d'émission de données (206), la brosse à dents (100) ayant un élément de nettoyage de dents (103) et un capteur (105), le moyen d'émission de données (206) comprenant un moyen pour émettre des données à partir du capteur (105), et le capteur étant un capteur de diagnostic (105) ;
une unité de calcul (303) configurée pour déduire un profil de santé d'un individu à l'aide de données obtenues à partir du moyen d'émission de données (206) de la brosse à dents (100) ; et
un dispositif d'affichage configuré pour afficher une représentation du profil de santé,
dans lequel la représentation du profil de santé comprend une indication quant à savoir si l'individu est soupçonné d'avoir un aspect de santé tel qu'un ou plusieurs parmi une gingivite, une parodontopathie, de la plaque dentaire, une halitose et une maladie cardiaque, sur la base des données obtenues ;
**caractérisé en ce que**
la représentation du profil de santé comprend une illustration graphique d'une cavité buccale divisée en une pluralité de régions différentes (501, 502, 503, 504, 505), la pluralité de régions différentes (501, 502, 503, 504, 505) comprenant une région de dents supérieure et une région de dents inférieure, et la sélection de l'un des aspects de santé amenant la représentation du profil de santé à fournir une indication quant à laquelle de la pluralité de régions différentes (501, 502, 503, 504, 505) de la cavité buccale est affectée par l'aspect de santé sélectionné ; et
**en ce que** la brosse à dents (100) comprend un capteur de position (104) actionnable pour détecter une position ou une orientation de la brosse à dents (100) de façon à indiquer si la brosse à dents (100) est maintenue avec le côté poils vers le haut ou le côté poils vers le bas ;
dans lequel le moyen d'émission de données (206) comprend un moyen pour émettre des données à partir du capteur de position (104) ; et
dans lequel l'indication quant à laquelle de la pluralité de régions différentes (501, 502, 503, 504, 505) de la cavité buccale qui comprennent une région de dents supérieure et une région de dents inférieure est affectée par l'aspect de santé, est obtenue à partir des données générées à l'origine par le capteur de position (104), lesquelles indiquent si la brosse à dents (100) est maintenue avec le côté poils vers le haut ou le côté poils vers le bas.

2. Système selon la revendication 1, dans lequel la représentation du profil de santé d'un individu est affichée sur la base de premières informations obtenues à partir de la brosse à dents (100), la représentation comprenant en outre une indication affichée d'un historique du profil de santé de l'individu sur la base de secondes informations obtenues antérieurement à partir de la brosse à dents (100).

3. Système selon la revendication 1, dans lequel les données sont reçues par l'intermédiaire d'Internet (304).

4. Système selon la revendication 1, dans lequel le dispositif d'affichage est séparé de la brosse à dents (100).

5. Système selon l'une quelconque des revendications 1 à 4, dans lequel la représentation du profil de santé comprend une indication d'un produit recommandé pour traiter l'aspect de santé.

6. Système selon la revendication 5, dans lequel le produit est un instrument d'hygiène buccale.

7. Système selon la revendication 5, dans lequel le produit est un dentifrice.

8. Système selon l'une quelconque des revendications 1 à 7, dans lequel l'indication comprend faire ressortir ou autrement distinguer visuellement les régions affectées.
